# EUROPEAN PATENT APPLICATION

(11) **EP 4 636 071 A1**
(43) Date of publication of application: **22.10.2025**
(21) Application number: 23903866.4
(22) Date of filing: 04.12.2023
(51) Int. Cl.: C12M 3/00, C12M 1/34, C12M 1/00, C12M 3/06

(54) **DECELLULARIZATION REACTION APPARATUS**

(30) Priority: 16.12.2022 KR 20220177587
(71) Applicant: POSCO Holdings Inc., Pohang-si, Gyeongsangbuk-do 37859 (KR); Research Institute of Industrial Science & Technology, Pohang-si, Gyeongsangbuk-do 37673 (KR); POSTECH Research and Business Development Foundation, Pohang-si, Gyeongsangbuk-do 37673 (KR)
(72) Inventor: LEE, Boram, Busan 48113 (KR); KIM, Hyejin, Gyeongsangbuk-do 37673 (KR); JANG, Jinah, Gyeongsangbuk-do 37673 (KR); AN, Hyeryeon, Gyeonggi-do 16397 (KR)
(74) Representative: Pfenning, Meinig & Partner mbB
(86) International application number: PCT/KR2023/019822
(87) International publication number: WO 2024/128663

(57) **Abstract**

A decellularization reaction apparatus is provided. A decellularization reaction apparatus according to the present invention comprises a mesh chamber for accommodating tissue of a xenogeneic organ to be decellularized therein, at least one reaction vessel for storing a decellularization solution, and a first reactor for in-situ analyzing various properties of the circulated decellularization solution while circulating the decellularization solution inside and outside the reaction vessel.

## Description

### [Technical Field]

The present invention relates to a decellularization reaction apparatus, and more particularly, to a decellularization reaction apparatus for bioink production.

### [Background Art]

Recently, with the global trend of aging populations, the number of patients with chronic diseases is increasing, and interest in artificial organ manufacturing technology using 3D bioprinting is growing.

Bio-ink is a term referring to ink material for printing artificial organs with a 3D bio printer and is manufactured based on extracellular matrix obtained after decellularizing xenogeneic organs.

Such a decellularization process of a xenogeneic organ is the most critical step in the production of bioink and refers to the process of removing all impurities and cells from the xenogeneic organ to prevent antigen-antibody reactions.

The decellularization process for application to 3D bioprinting technology involves immersing xenogeneic organs in a solution. Currently, no specialized apparatus for decellularization has existed, and simple laboratory glassware, such as beakers, has been used as a substitute for a dedicated device.

In addition, there was a problem that there was a difference in the physical properties and quality between the final products because there was no specific indicator that could be used to transfer the process of discharging the existing solution and injecting the next solution during the immersion process in various decellularization solutions.

### [DETAILED DESCRIPTION OF THE INVENTION]

### [Technical Problem]

The present invention provides a decellularization reaction apparatus configured to measure properties such as electrical conductivity, turbidity, and absorbance (UV-Vis) of a decellularization solution during the decellularization process, thereby enabling the derivation of indicators such as treatment time, reinjection or transition to the next solution, and termination of the decellularization process.

### [Technical Solution]

A decellularization reaction apparatus according to one embodiment of the present invention may comprise a mesh chamber for accommodating the tissue of the xenogeneic organ to be decellularized inside, and at least one reaction vessel for storing a decellularization solution to perform a decellularization reaction with a tissue of a xenogeneic organ inserted inside the mesh chamber.

In addition, the decellularization reaction apparatus may comprise a first reactor configured to discharge the decellularization solution undergoing the decellularization reaction inside the reaction vessel, recirculate the discharged decellularization solution back into the reaction vessel, and analyze various physical property of the circulating decellularization solution in-situ.

The decellularization reaction apparatus may further comprise a second reactor for extracting the decellularization solution undergoing the decellularization reaction inside the reaction vessel to the outside of the above reaction vessel and analyzing various property of the decellularization solution ex-situ.

Hereinafter, the first reactor may refer to a reactor to which an in-situ analysis device is applied, with analytical instruments such as a turbidity meter, an electrical conductivity meter, and an absorbance meter, etc. installed in the reactor tube.

Additionally, the second reactor may refer to a reactor in which an analysis device such as a turbidity meter, an electrical conductivity meter, and an absorbance meter are not installed in the reactor tube, and the method of measuring those properties is applied by sampling the solution and measuring it with an ex-situ method.

The first reactor may comprise a solution circulation part that is connected to be in communication between the reaction vessel and the cover of the reaction vessel and circulates the decellularization solution to the reaction vessel and the cover.

The first reactor may be installed in the solution circulation part and may comprise a measuring part for in-situ measuring various property of a decellularization solution undergoing a decellularization reaction circulated in the solution circulation part.

The mesh chamber may be coupled with a driving part for rotating the mesh chamber to be stirred within the reaction vessel.

The cover may be coupled with a lifting part for vertically lifting the cover relative to the upper part of the reaction vessel.

The mesh chamber may be formed in a cylindrical shape with an empty interior.

The mesh chamber may be manufactured or coated with a material that has chemical resistance depending on the property of the decellularization solution.

A outer surface of the mesh chamber may be arranged in a lattice form with a mesh.

The size of the mesh may be set to be smaller than the tissue of the xenogeneic organ to be decellularized.

A lid may be coupled to the upper part of the mesh chamber to cover the mesh chamber.

The reaction vessel may be made of a transparent material or has a transparent window to allow for internal observation.

The reaction vessel may be formed in a double jacket shape including an inner tube and an outer tube to control the temperature of the decellularization process.

The reaction vessel may have a solution outlet for discharging the decellularization solution.

A solution injection tube for injecting a decellularization solution into the interior of the reaction vessel may be connected to the above cover.

The driving part may comprise a rotational shaft coupled with the lid of the mesh chamber, and a driving motor installed on the cover of the reaction vessel and coupled with the rotational shaft to rotate the rotational shaft.

The solution circulation part may comprise a circulation tube connected between the cover and the solution outlet, for circulating the decellularization solution within the reaction vessel, and a transfer pump for quantitatively transferring the decellularization solution circulating in the circulation tube, which is installed in the circulation tube.

The circulation tube may be made of expandable a flexible tube.

The measuring part may comprise a absorbance analyzer, an electrical conductivity meter, and a turbidity meter for measuring the absorbance, electrical conductivity, and turbidity of the decellularization solution circulated in the circulation tube, respectively.

The measuring part may comprise a thermometer for measuring the temperature of the decellularization solution circulated in the circulation tube.

The lifting part may comprise a coupling plate coupled to the cover so as to be vertically movable, and a lifting cylinder coupled with the coupling plate and having a lifting rod for lifting the coupling plate.

The lifting cylinder may be connected to an air supply tube for supplying air into the lifting cylinder, and a regulator for adjusting the pressure of air supplied to the air supply tube may be installed in the air supply tube.

The second reactor may comprise a solution sampling tube for extracting and sampling the decellularized solution that is installed in the circulation tube and circulated through the circulation tube.

A solution sampling tube control valve may be installed in the solution sampling tube to control the opening and closing of the solution sampling tube.

A decellularization reaction apparatus according to another embodiment of the present invention may comprise a mesh chamber for accommodating the tissue of the xenogeneic organ to be decellularized inside and at least one reaction vessel for storing a decellularization solution to perform a decellularization reaction with a tissue of a xenogeneic organ inserted inside the mesh chamber.

In addition, the decellularization reaction device may comprise a second reactor for extracting a decellularization solution undergoing a decellularization reaction within the reaction vessel to the outside of the reaction vessel and analyzing various property of the decellularization solution ex-situ.

The decellularization reaction device may comprise a solution circulation part that is connected to be in communication between the reaction vessel and the cover of the reaction vessel and circulates the decellularization solution to the reaction vessel and the cover.

The mesh chamber may be coupled with a driving part for rotating the mesh chamber to be stirred within the reaction vessel.

The cover may be coupled with a lifting part for vertically lifting the cover relative to the upper part of the reaction vessel.

A outer surface of the mesh chamber may be arranged in a lattice form with a mesh.

The size of the mesh may be set to be smaller than the tissue of the xenogeneic organ to be decellularized.

The solution circulation part may comprise a circulation tube connected between the cover and the solution outlet, for circulating the decellularization solution within the reaction vessel, and a transfer pump for quantitatively transferring the decellularization solution circulating in the circulation tube, which is installed in the circulation tube.

The circulation tube may be made of expandable a flexible tube.

A solution sampling tube may be installed in the circulation tube to extract and sample the decellularization solution circulating through the circulation tube.

The reaction vessel may be arranged at a set interval on the base and supported by a support frame that is arranged in a vertical direction with respect to the base, and an alignment groove for positioning the reaction vessel and the cover may be arranged on the upper surface of the base.

A second elastic member may be coupled to the upper part of the first support frame to buffer the impact of the reaction vessel.

### [Advantageous Effects]

According to the embodiment of the present invention, by measuring the electrical conductivity, turbidity, and absorbance (UV-Vis) of the solution undergoing decellularization, it is possible to derive indicators such as the processing time of the decellularized solution, re-injection or transfer to the next solution, and the completion of the decellularization process, etc.

Additionally, by utilizing the reaction vessel, mesh chamber, and solution circulation structure, various physical properties of the solution (electrical conductivity, turbidity, and absorbance, etc.) can be analyzed during the decellularization process. Accordingly, the discharge, reinjection, and termination conditions of the decellularization solution can be determined, enabling the production of high-quality decellularized material while simultaneously ensuring material uniformity with excellent effectiveness.

### [Description of the Drawings]

FIG. 1 is a schematic configuration diagram of a first reactor of a decellularization reaction apparatus according to a first embodiment of the present invention.
FIG. 2 is a schematic side view of the first reactor of a decellularization reaction apparatus according to the first embodiment of the present invention.
FIG. 3 is a schematic configuration diagram of the mesh chamber of the first reactor in the decellularization reaction apparatus according to a first embodiment of the present invention.
FIG. 4 is a schematic side view of the second reactor of a decellularization reaction apparatus according to the second embodiment of the present invention
FIG. 5 is a graph showing the analysis results of the electrical conductivity of the decellularization solution measured during the porcine placenta decellularization process using the first reactor of the decellularization reaction apparatus according to a first embodiment of the present invention.
FIG. 6 is a graph showing the analysis results of the turbidity of the decellularization solution measured during the porcine placenta decellularization process using the first reactor of the decellularization reaction apparatus according to a first embodiment of the present invention.
FIG. 7 is a graph showing the analysis results of the absorbance (UV-Vis) of the decellularization solution measured during the porcine placenta decellularization process using the first reactor of the decellularization reaction apparatus according to a first embodiment of the present invention.
FIG. 8 is a photograph showing the state of the porcine placenta before and after the decellularization process using the first reactor of the decellularization reaction apparatus according to a first embodiment of the present invention.

### [BEST MODE FOR INVENTION]

Hereinafter, with reference to the attached drawings, an embodiment of the present invention will be described so that a person having ordinary skill in the art to which the present invention pertains can easily implement the invention. As can be easily understood by a person of ordinary skill in the technical field to which the present invention pertains, the embodiments described later may be modified in various forms without departing from the concept and scope of the present invention. As much as possible, identical or similar parts are represented in the drawing using the same drawing symbol.

The technical terms used below are merely for the purpose of mentioning specific embodiments and are not intended to limit the present invention. The singular forms used here comprise plural forms unless the phrases clearly indicate the opposite meaning. The meaning of ' comprising' used in the specification specifies certain characteristics, areas, integers, stages, actions, elements, and/or components, and does not exclude the presence or addition of other specific characteristics, areas, integers, stages, actions, elements, components, and/or groups.

All terms including technical and scientific terms used below have the same meaning as understood generally by a person of ordinary skill in the technical field to which the invention pertains. The terms defined in the dictionary are interpreted as having meanings that correspond to the relevant technical literature and the currently disclosed content, and unless otherwise defined, are not interpreted in an ideal or very formal sense.

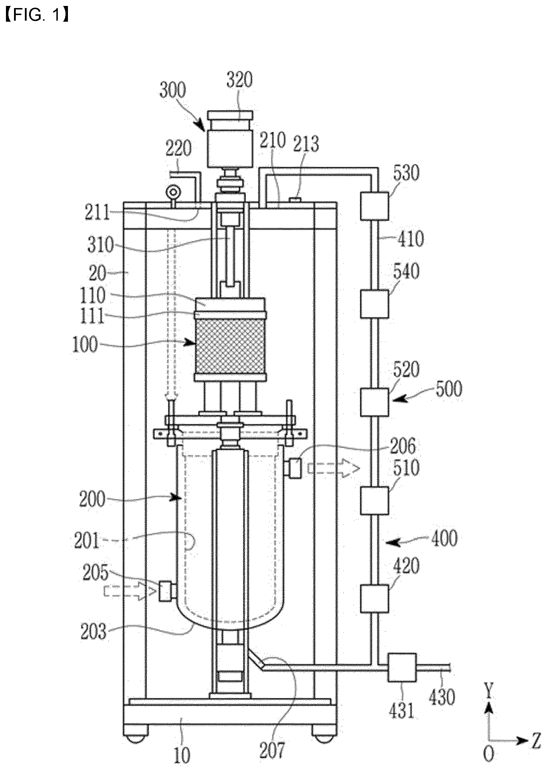

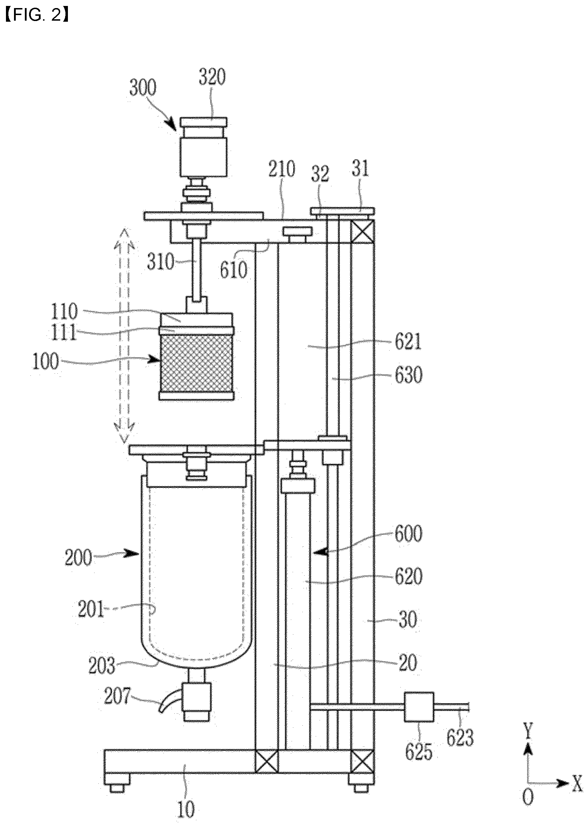

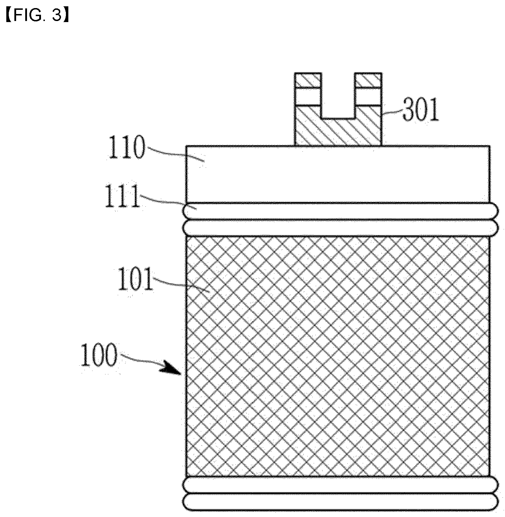

FIG. 1 is a schematic configuration diagram of a first reactor of a decellularization reaction apparatus according to a first embodiment of the present invention, and FIG. 2 is a schematic side view of the first reactor of a decellularization reaction apparatus according to the first embodiment of the present invention, and FIG. 3 is a schematic configuration diagram of the mesh chamber of the first reactor in the decellularization reaction apparatus according to a first embodiment of the present invention.

Referring to FIG. 1 to 3, a decellularization reaction apparatus according to the first embodiment of the present invention may comprise a mesh chamber 100, a reaction vessel 200, and a first reactor.

The mesh chamber 100 has a predetermined size and shape and may accommodate the tissue of the xenogeneic organ to be decellularized inside.

Additionally, the reaction vessel 200 may have at least one mesh chamber 100 inserted therein and may store a decellularization solution for performing a decellularization reaction with the tissue of the xenogeneic organ within the mesh chamber 100.

The first reactor may discharge the decellularization solution, which is undergoing a decellularization reaction with the xenogeneic organ tissue inside the reaction vessel 200, from the reaction vessel 200, and then recirculate it back into the interior of the reaction vessel 200, while analyzing various physical properties of the circulating first decellularization solution in-situ.

Additionally, the first reactor may comprise a driving part 300, a solution circulation part 400, and a measuring part 500.

The driving unit 300 may be coupled to the mesh chamber 100 and may rotate the mesh chamber 100 to enable agitation within the reaction vessel 200.

Additionally, the solution circulation unit 400 may be connected in fluid communication between the reaction vessel 200 and the cover 210 that covers the reaction vessel 200, and may circulate the decellularization solution between the reaction vessel 200 and the cover 210.

The measuring part 500 is installed in the solution circulation part 400 and may measure and analyze various physical properties of the decellularization solution, which is undergoing a decellularization reaction and circulating through the solution circulation part 400, in-situ.

A cover 210 may be placed on the upper part of the reaction vessel 200 to cover the reaction vessel 200.

Additionally, it may comprise a lifting part 600 for lifting the cover 300 in a vertical direction (Y direction in FIG. 1) so as to be combined with the cover 210 and to be able to combine or separate the cover 210 from the reaction vessel 200.

The mesh chamber 100 may be formed in a hollow shape with an empty interior so as to accommodate tissue of a xenogeneic organ to be decellularized inside.

The mesh chamber 100 may have a cylindrical shape or the like to allow for easy rotation by the driving part 300.

In addition, when the mesh chamber 100 is made in a square shape, the tissue of the xenogeneic organ may become caught or remain at the sharp corners when the mesh chamber 100 rotates, and the tissue of the xenogeneic organ may not be stirred uniformly. Therefore, the reason for making the mesh chamber 100 in a cylindrical shape is to stir the tissue of the xenogeneic organ uniformly and efficiently.

The external surface of the mesh chamber 100 is arranged in a grid shape with the mesh 101, and the mesh 101 may have a set size, for example, a size of about 1 mm, for the decellularization reaction of the tissues of xenogeneic organs accommodated within the mesh chamber 100.

The mesh chamber 100 may be configured to be larger than the mesh 100A of the second embodiment, and may be configured as a large type capable of accommodating, for example, 500 g (maximum 1 kg) of a xenogeneic organ to be decellularized.

Additionally, the size of the mesh 101 may be set to be slightly smaller size than the tissue of the xenogeneic organ so that the tissue of the xenogeneic organ accommodated in the mesh chamber 100 does not escape from the mesh 101 during the decellularization process.

The decellularization reaction apparatus may comprise a lid 110 that is detachably connected to the upper part of the mesh chamber 100 and covers the mesh chamber 100.

The lid 110 may have a buckle 111 shape or the like, to be easily connected to the mesh chamber 100.

The driving part 300 may be coupled to the upper part of the lid 110.

The mesh chamber 100 may be manufactured or coated with materials that have good chemical resistance, such as Teflon, depending on the properties of the decellularization solution.

Additionally, the reaction vessel 200 may be made of a transparent material or may have a transparent window (not shown) so that the progress of the decellularization process performed within the reaction vessel 200 may be visually observed from outside the reaction vessel 200.

The reaction vessel 200 may be arranged at a set interval on the base 10 and supported by a first support frame 20 placed in a vertical direction (Y direction in FIG. 1) with respect to the base 10.

The reaction vessel 200 may be made of a material that is corrosion resistant to the decellularization solution to be used.

Additionally, the reaction vessel 200 may have a double jacket shape including an inner tube 201 and an outer tube 203 to control the temperature of the decellularization process.

The outer tube 203 may be equipped with a refrigerant inlet 205 for injecting refrigerant into the space between the outer tube 203 and the inner tube 201, and a refrigerant outlet 206 for discharging the refrigerant to the outside.

The reaction vessel 200 may have a solution outlet 207 at its lower portion for discharging the decellularization solution.

The cover 210 is made of a cushioning material such as rubber, etc. to seal the reaction vessel 200 while buffering the impact when it is lowered by the lifting part 600 and comes into contact with the upper part of the reaction vessel 200, and may have an O-ring shape, etc for sealing.

The cover 210 may be provided with at least one injection port 211 to which a solution injection tube 220 is connected for injecting various decellularization solutions into the interior of the reaction vessel 200

Various decellularization solutions may be composed of, for example, ultrapure water, organic solvents, etc., and each decellularization solution may be injected through each injection port 211, and a pump (not shown) for quantitatively supplying each decellularization solution may be installed in a tube (not shown) connected to each injection port 211.

Additionally, the cover 210 may be connected to the circulation part 400, allowing the decellularization solution circulating through the circulation part 400 to be injected into the interior of the reaction vessel 200.

A level measuring device 213, such as an ultrasonic method, for measuring the level of the decellularization solution in the reaction vessel 200 may be installed in the cover 210.

The driving part 300 may comprise a rotational shaft 310 coupled with the lid 110 of the mesh chamber 100, and a driving motor 320 installed on the cover 210 of the reaction vessel 200 and coupled with the rotational shaft 310 to rotate the rotational shaft 310.

The driving motor 320 may be formed of a motor of magnetic type, etc., so as to easily rotate the rotational shaft 310.

The rotational shaft 310 may be arranged in a vertical direction (Y-direction in FIG. 2) with respect to the upper part of the lid 110, positioned at the center of the upper part of the lid 110, and may be coupled to the lid 110 through fastening means such as a bolt 301.

The rotational shaft 310 may be rotated, for example, in a clockwise or counterclockwise direction by the driving motor 320, thereby rotating the mesh chamber 100 to agitate the tissue of the xenograft target for decellularization accommodated in the mesh chamber 100, thereby inducing smooth decellularization by the decellularization solution injected into the reaction vessel 200.

When the mesh chamber 100 is rotated and stirred at a predetermined rotation speed (e.g., 0 to 300 rpm), the stirring may be done alternately from the forward direction (clockwise) to the reverse direction (counterclockwise) to prevent overload of the drive motor 320 and to prevent clumping between xenogeneic organs. In this case, a stop time can be set in the middle.

The circulation part 400 may comprise a circulation tube 410 and a transfer pump 420.

The circulation tube 410 is connected between the cover 210 and the solution outlet 207 and may circulate the decellularization solution in the reaction vessel 200.

Additionally, the transfer pump 420 is installed in the circulation tube 410 and may quantitatively transfer the decellularization solution circulating in the circulation tube 410.

The circulation tube 410 may be made of a flexible tube or the like to allow for easy expansion, contraction, and deformation in shape during the vertical movement of the cover 210 by the lifting part 600.

Additionally, a waste liquid discharge tube 430 for discharging the waste liquid of the decellularization solution is connected to the circulation tube 410, and a waste liquid discharge tube control valve 431 for controlling discharge of the decellularization solution may be installed in the waste liquid discharge tube 430.

The measuring part 500 may comprise an absorbance analyzer 510 for analyzing the absorbance (UV-Vis) of the decellularization solution circulating in the circulation tube 410.

Additionally, the measuring part 500 may comprise an electrical conductivity meter 520 for measuring the electrical conductivity of the decellularization solution circulated in the circulation tube 410.

The measuring part 500 may comprise a turbidity meter 530 for measuring the turbidity of the decellularization solution circulated in the circulation tube 410.

The measuring part 500 may comprise a thermometer 540 for measuring the temperature of the decellularization solution circulated in the circulation tube 410.

The lifting part 600 may comprise a coupling plate 610 coupled integrally with the cover 210 so as to be vertically movable, and a lifting cylinder 620 coupled to the lower end of coupling plate 610 and having a lifting rod 621 for lifting the coupling plate 610.

The lifting cylinder 620 may be installed and supported on the upper surface of the base 10 in a vertical direction (Y direction in FIG. 2) relative to the base 10.

The lifting cylinder 620 may be connected to an air supply tube 623 for supplying air into the lifting cylinder 620, and a regulator 625 for adjusting the pressure of air supplied to the air supply tube 623 may be installed in the air supply tube 623.

Additionally, a second support frame 30 may be installed on the base 10 and is arranged parallel to the first support frame 20 and supported by the base 10.

The second support frame 30 is connected to the connecting frame 31, and a guide rod 630 for guiding the vertical movement of the lifting rod 621 may be coupled.

Hereinafter, with reference to FIG. 1 to 3, the operation of the decellularization reaction apparatus according to the first embodiment of the present invention will be described.

First, the tissue of the xenogeneic organ to be decellularized and the decellularization solution for performing the decellularization reaction with the tissue of the xenogeneic organ are prepared.

In order to inject the tissue of the xenogeneic organ to be decellularized and the decellularization solution into the mesh chamber 100 and the reaction vessel 200, respectively, as shown in FIG. 1, the lid 110 is opened from the mesh chamber 100, and the cover 210 is opened from the reaction vessel 200.

In this state, the tissue of the xenogeneic organ to be decellularized is injected into the inside of the mesh chamber 100, the upper part of the mesh chamber is covered with a lid 110, and tthe lid 110 is coupled to the mesh chamber 100 using the buckle 111.

And, when the lifting rod 621 of the lifting cylinder 620 in the lifting part 600 is actuated to retract, the cover 210 covers the upper part of the reaction vessel 200 and seals the reaction vessel 200 as the lifting rod 621 is lowered to a predetermined length.

As such, when the cover 210 seals the reaction vessel 200, the mesh chamber 100 is inserted into the interior of the reaction vessel 200.

At this time, the decellularization solution is injected into the interior of the reaction vessel 200 through the injection port 211 installed on the cover 210 and connected to the solution injection tube 220.

Then, when the injection of the decellularization solution into the reaction vessel 200 is completed, the drive motor 320 of the drive unit 300 is driven to rotate the rotation shaft 310 at a predetermined speed in the clockwise or counterclockwise direction, thereby rotating and agitating the mesh chamber 100.

As such, as the mesh chamber 100 is rotated and agitated, the tissue of the xenogeneic organ in the mesh chamber 100 is agitated and undergoes a decellularization reaction with the decellularization solution in the reaction vessel 200.

At this time, the tissue of the xenogeneic organ is stirred by the rotation of the mesh chamber 100 and the decellularization solution is moved vertically from the cover 210 into the reaction vessel 200 through the circulation tube 410, so that the tissue of the xenogeneic organ and the decellularization solution are well mixed, thereby obtaining a decellularization solution with a homogeneous concentration in real time.

In this state, the transfer pump 420 is operated to circulate the decellularization solution undergoing decellularization reaction within the reaction vessel 200 through the circulation tube 410 at a predetermined speed.

The physical properties of the decellularization solution circulating from the solution outlet 207 of the reaction vessel 200 to the cover 210 through the circulation tube 410 may be measured in real time by the measuring part 500 installed on the circulation tube 410.

That is, the absorbance (UV-Vis) of the decellularization solution is measured and analyzed by the absorbance analyzer 510, the electrical conductivity is measured by the electrical conductivity meter 520, the turbidity is measured by the turbidity meter 530, and the temperature of the decellularization solution is measured by the thermometer 540.

Additionally, by measuring the decellularization solution of a homogeneous concentration, various physical properties of the decellularization solution-such as absorbance (UV-Vis), electrical conductivity, and turbidity-can be easily analyzed, enabling real-time and accurate monitoring of the progress of the decellularization process.

### (embodiment)

Hereinafter, as an example of a xenogeneic organ to be decellularized, a decellularization experiment using a porcine placenta will be described.

First, 500g of pretreated porcine placenta, from which foreign substances have been removed, is placed into the mesh chamber 100 inside the decellularization reaction vessel 200, and the lid 110 of the mesh chamber 100 is closed. In the case of pig placenta, decellularization is possible at room temperature of 20°C, so the temperature is not specifically set.

Then, gas (air) is pressurized in the lifting cylinder 620 and the reaction vessel 200 is sealed by covering it with the cover 210.

After injecting the decellularization solution into the reaction vessel 200, the speed of the rotation shaft 310 driven by the drive motor 320 is set to 80 rpm, and the mesh chamber 100 is rotated and stirred with settings of, for example, 10 seconds clockwise, 8 seconds stop time, and 10 seconds counterclockwise.

At the same time, the decellularization solution inside the reaction vessel 200 is circulated to the circulation tube 410 using a quantitative transfer pump 420. At this time, the circulation speed of the decellularization solution is set to 1,000 ml/min.

The decellularization solution in the reaction vessel 200 may obtain the homogeneous concentration through circulation by the transfer pump 420 and agitation by the rotation of the mesh chamber 100.

Thereafter, in the case of the reaction vessel 100 according to the first embodiment, data on various physical properties of the decellularization solution during the decellularization process may be analyzed in real time using the absorbance (UV-Vis) analyzer 510, the electrical conductivity meter 520, and the turbidity meter 530 installed in the circulation tube 410 (see Figure 1).

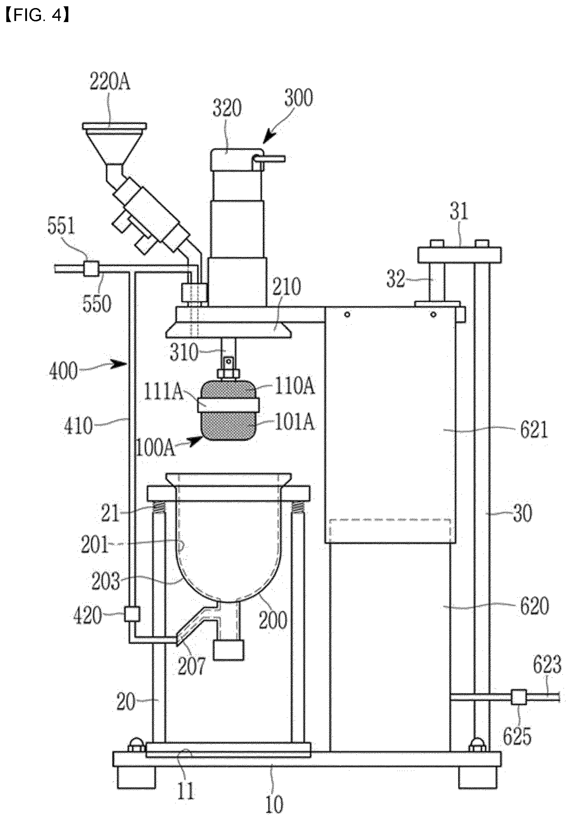

Additionally, in the case of the reaction vessel 100A according to the second embodiment, various physical properties of the decellularization solution may be analyzed through sampling of the decellularization solution using a solution sampling tube 550 (see FIG. 4) installed on the circulation tube 410.

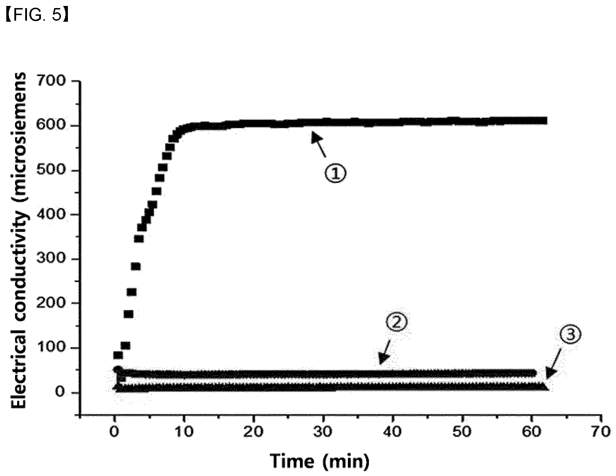

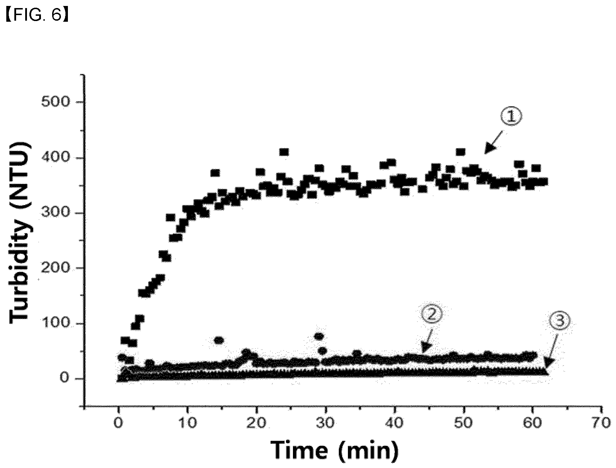

FIG. 5 is a graph showing the analysis results of the electrical conductivity of the decellularization solution measured during a porcine placenta decellularization process, and FIG. 6 is a graph showing the analysis results of the turbidity of the decellularization solution measured during a porcine placenta decellularization process.

FIGS. 5 and 6 show the results of analyzing the decellularization solution while performing three ultrapure water processes. Here, ① refers to the case of performing the first ultrapure water process, ② refers to the case of performing the second ultrapure water process, and ③ refers to the case of performing the third ultrapure water process.

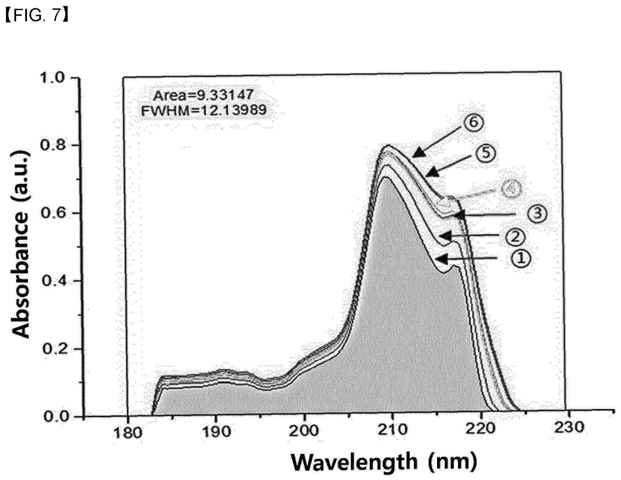

FIG. 7 is a graph showing the analysis results of the absorbance (UV-Vis) of the decellularization solution measured during the porcine placenta decellularization process.

In Fig. 7, ①, ②, ③, ④, ⑤, and ⑥ are data of absorbance over time during the first ultrapure water process.

Here, ① is measurement data after 10 minutes, ② is measurement data after 20 minutes, ③ is measurement data after 30 minutes, ④ is measurement data after 40 minutes, ⑤ is measurement data after 50 minutes, and ⑥ is measurement data after 60 minutes.

During the decellularization process, as time passes, the physical property values in the reactor tend to initially increase and then gradually decrease, when the decellularization solution is discharged and the same decellularization solution is re-injected, it can be confirmed that the properties change significantly, as shown in ①-②-③ of Figs. 5 and 6.

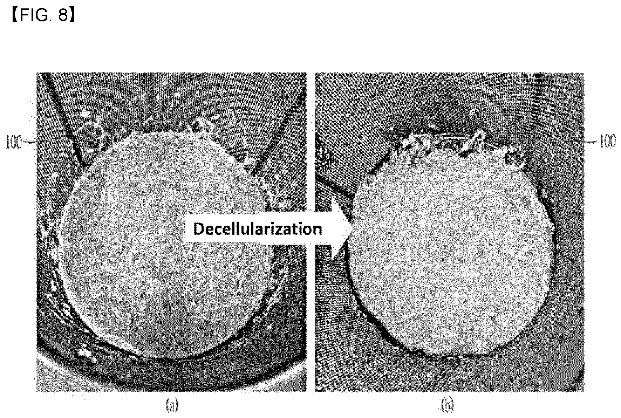

In this way, by monitoring various physical property values of the decellularization solution, it is possible to determine whether reinjection of each decellularization solution is necessary or the completion conditions of the process, and this procedure induces decellularization to proceed stably, as shown in Fig. 8. Therefore, the final decellularized placental tissue material is of high quality and homogeneity.

### (Test results for performance, etc.)

When evaluating the quality of bioink, the analysis of DNA content and collagen content is the most fundamental.

[Table 1] shows the results comparing the porcine placental DNA content before and after using the decellularization reaction apparatus. Each porcine placenta was supplied from a different source, which resulted in large differences in DNA content among porcine placentas.

**[Table 1] Comparison of DNA content in porcine placental tissue before and after using the decellularization reaction apparatus**

| [Table 1] | | | |
|---|---|---|---|
| DNA Content | Placenta A (Supplier A) | Placenta B (Supplier B) | Placenta C (Supplier C) |
| Before decellularization | 87.2 ng/mg | 77.5 ng/mg | 244.8 ng/mg |
| After decellularization | 0.37 ng/mg | 0.48 ng/mg | 0.46 ng/mg |

As shown in the above results, the DNA content, which ranged from 87 to 244 ng/mg before using the decellularization reaction apparatus, was found to an extremely low level of 0.37 to 0.48 ng/mg after using the decellularization reaction apparatus.

It is significant that not only was the DNA content reduced, but high-quality and homogeneous results were achieved despite the use of placentas from pigs raised in different locations.

**[Table 2] Comparison of collagen content in porcine placental tissue before and after use of the decellularization reaction apparatus**

| [Table 2] | | | |
|---|---|---|---|
| Collagen Content | Placenta A (Supplier A) | Placenta B (Supplier B) | Placenta C (Supplier C) |
| Before decellularization | 10.1 µg/mg | 8.9 µg/mg | 6.5 µg/mg |
| After decellularization | 22.4 µg/mg | 23.2 µg/mg | 22.3µg/mg |

[Table 2] shows the results comparing the porcine placental collagen content before and after using the decellularization apparatus. As shown in the above results, collagen was well preserved at a level of 22-23 µg/mg even after using the decellularization reaction apparatus.

Therefore, after using the decellularization reaction apparatus, the DNA content in the tissue was reduced to an extremely low level, and the collagen within the tissue was well preserved, indicating that a high-quality bioink material was produced.

FIG. 4 is a schematic side view of the second reactor of a decellularization reaction apparatus according to the second embodiment of the present invention.

The second reactor of the decellularization reaction apparatus according to the second embodiment of the present invention is the same as the first reactor of the decellularization reaction apparatus according to the first embodiment, except for the matters specifically described below, and therefore, detailed descriptions thereof will be omitted.

Referring to FIG. 4, the decellularization reaction apparatus according to the second embodiment of the present invention may comprise a second reactor for extracting the tissue of the xenogeneic organ to be decellularized and the decellularization solution undergoing the decellularization reaction inside the reaction vessel 200 to the outside of the reaction vessel 200 to analyze various properties of the decellularization solution ex-situ.

The second reactor may comprise a solution sampling tube 550 installed on the circulation tube 410 of the circulation part 400 and for extracting and sampling the decellularization solution circulated through the circulation tube 410.

After placing the decellularization solution sampled in the solution sampling tube 550 in a small container (not shown) such as a vial, various physical properties (absorbance, electrical conductivity, turbidity, etc.) of the decellularization solution contained in the small container can be measured and analyzed by ex-situ.

A solution sampling tube control valve 551 may be installed on the solution sampling tube 550 to control the opening and closing of the solution sampling tube 550.

The mesh chamber 100A may be configured to be smaller than the mesh chamber 100 of the first embodiment, that is, it may be configured as a small type that can accommodate a maximum amount of xenogeneic organ to be decellularized less than 25 g. and accordingly, the mesh 101A of the mesh chamber 100A may also be set to a smaller size, that is, less than 1 mm.

The reaction vessel 200 may be placed at a set interval on the base 10 and supported by a first support frame 20 placed in a vertical direction (Y direction in FIG. 1) with respect to the base 10.

The reaction vessel 200 and the first support frame 20 may be configured to be movable and detachable for ease of cleaning, and an alignment groove 11 may be arranged on the upper surface of the base 10 for positional alignment of the reaction vessel 200 and the cover 210.

An elastic member 21 may be coupled to the upper end of the first support frame 20 to cushion the impact of the reaction vessel 200 that occurs when the cover 210 is lowered to seal the reaction vessel 200.

Although the present disclosure has been described above through preferred embodiments, it will be readily understood by those skilled in the art that the present invention is not limited thereto and that various modifications and variations are possible without departing from the scope of the claims set forth below.

### (Explanation of symbols)

100: mesh chamber
200: reaction vessel

## Claims

1. A decellularization reaction apparatus, comprises;
a mesh chamber for accommodating the tissue of the xenogeneic organ to be decellularized inside,
at least one reaction vessel for storing a decellularization solution to perform a decellularization reaction with a tissue of a xenogeneic organ inserted inside the mesh chamber, and
a first reactor configured to discharge the decellularization solution undergoing the decellularization reaction inside the reaction vessel, recirculate the discharged decellularization solution back into the reaction vessel, and analyze various physical property of the circulating decellularization solution in-situ.

2. The decellularization reaction apparatus of claim 1, further comprises:
a second reactor for extracting the decellularization solution undergoing the decellularization reaction inside the reaction vessel to the outside of the above reaction vessel and analyzing various property of the decellularization solution ex-situ.

3. The decellularization reaction apparatus of claim 2, wherein:
the first reactor comprises a solution circulation part that is connected to be in communication between the reaction vessel and the cover of the reaction vessel and circulates the decellularization solution to the reaction vessel and the cover.

4. The decellularization reaction apparatus of claim 3, wherein:
the first reactor is installed in the solution circulation part and comprises a measuring part for in-situ measuring various property of a decellularization solution undergoing a decellularization reaction circulated in the solution circulation part.

5. The decellularization reaction apparatus of claim 4, wherein:
the mesh chamber is coupled with a driving part for rotating the mesh chamber to be stirred within the reaction vessel.

6. The decellularization reaction apparatus of claim 5, wherein:
the cover is coupled with a lifting part for vertically lifting the cover relative to the upper part of the reaction vessel.

7. The decellularization reaction apparatus of claim 6, wherein:
the mesh chamber is formed in a cylindrical shape with an empty interior.

8. The decellularization reaction apparatus of claim 6, wherein:
the mesh chamber is manufactured or coated with a material that has chemical resistance depending on the property of the decellularization solution.

9. The decellularization reaction apparatus of claim 7, wherein:
a outer surface of the mesh chamber is arranged in a lattice form with a mesh.

10. The decellularization reaction apparatus of claim 9, wherein:
the size of the mesh is set to be smaller than the tissue of the xenogeneic organ to be decellularized.

11. The decellularization reaction apparatus of claim 10, wherein:
a lid is coupled to the upper part of the mesh chamber to cover the mesh chamber.

12. The decellularization reaction apparatus of any one of claim 1 to claim 11, wherein:
the reaction vessel is made of a transparent material or has a transparent window to allow for internal observation.

13. The decellularization reaction apparatus of claim 12, wherein:
the reaction vessel is formed in a double jacket shape including an inner tube and an outer tube to control the temperature of the decellularization process.

14. The decellularization reaction apparatus of claim 13, wherein:
the reaction vessel has a solution outlet for discharging the decellularization solution.

15. The decellularization reaction apparatus of claim 14, wherein:
a solution injection tube for injecting a decellularization solution into the interior of the reaction vessel is connected to the above cover.

16. The decellularization reaction apparatus of claim 11, wherein:
the driving part, comprises:
a rotational shaft coupled with the lid of the mesh chamber,
a driving motor installed on the cover of the reaction vessel and coupled with the rotational shaft to rotate the rotational shaft.

17. The decellularization reaction apparatus of claim 14, wherein:
the solution circulation part, comprises:
a circulation tube connected between the cover and the solution outlet, for circulating the decellularization solution within the reaction vessel, and
a transfer pump for quantitatively transferring the decellularization solution circulating in the circulation tube, which is installed in the circulation tube.

18. The decellularization reaction apparatus of claim 17, wherein:
the circulation tube is made of expandable a flexible tube.

19. The decellularization reaction apparatus of claim 18, wherein:
the measuring part comprises a absorbance analyzer, an electrical conductivity meter, and a turbidity meter for measuring the absorbance, electrical conductivity, and turbidity of the decellularization solution circulated in the circulation tube, respectively.

20. The decellularization reaction apparatus of claim 19, wherein:
the measuring part comprises a thermometer for measuring the temperature of the decellularization solution circulated in the circulation tube.

21. The decellularization reaction apparatus of claim 6, wherein:
the lifting part, comprises:
a coupling plate coupled to the cover so as to be vertically movable, and
a lifting cylinder coupled with the coupling plate and having a lifting rod for lifting the coupling plate.

22. The decellularization reaction apparatus of claim 21, wherein:
the lifting cylinder is connected to an air supply tube for supplying air into the lifting cylinder, and
a regulator for adjusting the pressure of air supplied to the air supply tube is installed in the air supply tube.

23. The decellularization reaction apparatus of claim 2, wherein:
the second reactor, comprises:
a solution sampling tube for extracting and sampling the decellularized solution that is installed in the circulation tube and circulated through the circulation tube.

24. The decellularization reaction apparatus of claim 23, wherein:
a solution sampling tube control valve is installed in the solution sampling tube to control the opening and the closing of the solution sampling tube.

25. A decellularization reaction apparatus, comprises:
a mesh chamber for accommodating the tissue of the xenogeneic organ to be decellularized inside,
at least one reaction vessel for storing a decellularization solution to perform a decellularization reaction with a tissue of a xenogeneic organ inserted inside the mesh chamber, and
a second reactor for extracting a decellularization solution undergoing a decellularization reaction within the reaction vessel to the outside of the reaction vessel and analyzing various property of the decellularization solution ex-situ.

26. The decellularization reaction apparatus of claim 25, comprises:
a solution circulation part that is connected to be in communication between the reaction vessel and the cover of the reaction vessel and circulates the decellularization solution to the reaction vessel and the cover.

27. The decellularization reaction apparatus of claim 26, wherein:
the mesh chamber is coupled with a driving part for rotating the mesh chamber to be stirred within the reaction vessel.

28. The decellularization reaction apparatus of claim 27, wherein:
the cover is coupled with a lifting part for vertically lifting the cover relative to the upper part of the reaction vessel.

29. The decellularization reaction apparatus of claim 25, wherein:
a outer surface of the mesh chamber is arranged in a lattice form with a mesh.

30. The decellularization reaction apparatus of claim 29, wherein:
the size of the mesh is set to be smaller than the tissue of the xenogeneic organ to be decellularized.

31. The decellularization reaction apparatus of any one of claim 25 to claim 30, wherein:
the solution circulation part, comprises:
a circulation tube connected between the cover and the reaction vessel, for circulating the decellularization solution within the reaction vessel, and
a transfer pump for quantitatively transferring the decellularization solution circulating in the circulation tube, which is installed in the circulation tube.

32. The decellularization reaction apparatus of claim 31, wherein:
the circulation tube is made of expandable a flexible tube.

33. The decellularization reaction apparatus of claim 32, wherein:
the second reactor comprises a solution sampling tube for extracting and sampling the decellularized solution that is installed in the circulation tube and circulated through the circulation tube.

34. The decellularization reaction apparatus of claim 31, wherein:
the reaction vessel is arranged at a set interval on the base and supported by a first support frame that is arranged in a vertical direction with respect to the base, and
an alignment groove for positioning the reaction vessel and the cover is arranged on the upper surface of the base.

35. The decellularization reaction apparatus of claim 34, wherein:
an elastic member is coupled to the upper part of the first support frame to buffer the impact of the reaction vessel.
